Europäisches Patentamt

European Patent Office

Office européen des brevets

(11) Publication number: **0 240 593 B1**

# EUROPEAN PATENT SPECIFICATION

(45) Date of publication of patent specification: **19.05.93**    (51) Int. Cl.⁵: **A61N 1/30, A61M 37/00**

(21) Application number: **86110164.0**

(22) Date of filing: **24.07.86**

Divisional application 92119093.0 filed on 24/07/86.

(54) **Improved transdermal drug applicator and electrodes therefor.**

(30) Priority: **14.03.86 US 839523**

(43) Date of publication of application:
**14.10.87 Bulletin 87/42**

(45) Publication of the grant of the patent:
**19.05.93 Bulletin 93/20**

(84) Designated Contracting States:
**AT BE CH DE FR GB IT LI LU NL SE**

(56) References cited:
**EP−A− 0 147 524
EP−A− 0 178 601
WO−A− 86/07269**

(73) Proprietor: **Drug Delivery Systems Inc.
292 Madison Avenue
New York New York 10017(US)**

(72) Inventor: **Sibalis, Dan
268 Hallock Road
Stony Brook N.Y. 11790(US)**

(74) Representative: **Patentanwälte Ruff, Beier,
Schöndorf und Mütschele
Neckarstrasse 50
W−7000 Stuttgart 1 (DE)**

## Description

This invention relates to a transdermal drug applicator according to the first part of claim 1.

Such a drug applicator is already known (EP-A-0 147 524). Within this known applicator an electrically conductive adhesive bonds the lip of the cover to the skin.

SUMMARY OF THE INVENTION

Transdermal drug applicators embody various electrode constructions. However, since the overall size of the applicator should be minimized for cost effectiveness and aesthetics and the dosage capability desirably maximized, it is an object of the present invention to provide improved applicators as well as improved electrode constructions which are applicable to both the active and inactive electrodes. In addition, it is another object of a preferred embodiment of the invention to provide drug applicators which employ only active electrodes, or with different drugs in each electrode structure, or with the active and inactive electrodes of different sizes. Moreover, in a preferred embodiment the invention further pertains to drug applicators incorporating three electrodes which may operate to deliver drugs independently of each other using separate mass transfer phenomenas, such as electrophoresis and electroosmosis or endosmose.

These and other objects and advantages of the invention will become more apparent from a reading of the following detailed description of the preferred modifications and embodiments of the invention.

Within the drawings fig. 1 to 4 show how a drug applicator works in prinicipal. Some figures, in particular fig. 2, don't show all features of the invention.

BRIEF DESCRIPTION OF THE DRAWINGS

Figure 1 is a perspective view taken from above or the top of a drug applicator embodying the principles of the invention;

Figure 2 is a cross-sectional view taken along the line 2-2 of Figure 1 and showing the applicator mounted on skin (no part of the invention);

Figure 3 is an electrical schematic of the circuit incorporated in the drug applicator shown in Figures 1-2;

Figure 4 is an alternate arrangement for the circuit shown in Figure 3;

Figure 5 is a perspective view taken from above or the top of an alternate drug applicator embodying spaced apart electrodes in a side by sidle fashion (no part of the invention);

Figure 6 is a partial cross-sectional view taken along the line 6-6 of Figure 5;

Figure 7 is a further electrical schematic illustrative of a circuit embodying three active electrodes, such as the active electrode construction exhibited in Figure 6;

Figure 8 is a bottom plan view of yet another drug applicator structure incorporating a plurality of high tack spots across the electrodes employed and the drug reservoir or matrix;

Figure 9 is a partial cross-sectional view taken along the line 9-9 of Figure 8;

DESCRIPTION OF THE PREFERRED EMBODIMENTS

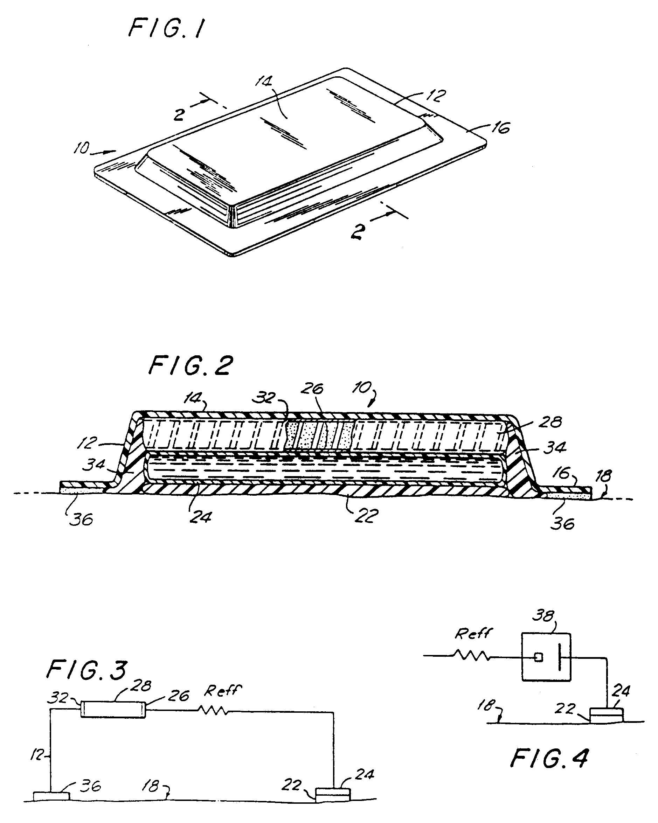

Referring to Figures 1 and 2, applicator 10 consists of an outer cover 12 having a raised portion 14 and a lip 16 along the outer periphery. It is understood that applicator 10 can have any convenient shape or size, for example, square, rectangular, oval, circular, or tailored for a specific location on the skin, as long as this is a raised central portion to accommodate the rest of the electrophoresis unit to be described and the lip along its periphery.

As seen in Fig. 2, where applicator 10 is mounted on the surface of skin 18 of a patient, enclosed within the raised portion 14 of cover 12 are several layers to be described. The first layer is a microporous or semi-permeable membrane 22 through which the medicament migrates to be deposited on skin 18. As will be noted from the following discussion, membrane 22 may not be needed depending on the nature of the reservoir for the medicament.

The second layer consists of a flexible pouch or reservoir 24 containing the drug to be administered. As is understood in the art resevoir 24 can be a pouch containing the drug of choice in solution or suspension, the walls of which are sufficiently dense to prevent leakage of the drug under ambient conditions, but sufficiently porous to permit migration of the charged particles or ions under the influence of the electric field imposed. It should be noted that it would be appropriate to employ the microporous membrane 22

2

when leakage under ambient conditions could occur, for example, as a result of packing of the applicators for shipment or storage, fluctuating temperatures, and possibly puncture of the reservoir. Also, the use of the membrane 22 could depend in large measure on the nature of the medicament involved. In the alternative, reservoir 24 can consist of porous material in which the drug is impregnated rather than a pouch containing the liquid medicament.

The third or next layer above reservoir 24 is an extended contact 26 which could be incorporated as one face of battery 28 which is the next layer. Contact 26 could be any suitable conductive material, preferably conformable to permit applicator 10 to be curved or bent to conform to the shaped surface of the skin. Suitable materials of this type are well known in the art and include electrically conductive polymers, preferably non–ionic. Carbon loaded or surface metalized plastics are also available for such use.

Battery 28 comprising the next layer can be made up of a group of cells internally connected in series to obtain the desired voltage necessary to obtain the electrophoretic action with the particular medicament. Orientation of battery 28 would depend on whether the charged (ionic) particles of the drug of choice are positive or negative. If the particles are negatively charged in solution or suspension then contact 26 would be connected to the negative side of battery 28 as the skin will then be positive with respect to that contact and will attract the ions. With regard to battery 28, it should be noted that any conventional miniaturized battery cells now generally available can be employed, arranged and connected in series to obtain the desired operating voltage. In addition, the technology now exists for batteries which are made up of very thin, flexible sheets of a conductive polymer with high surface areas relative to thickness to provide adequate current densities. One such so–called plastic battery is described in "Batteries Today", Autumn 1981, pages 10, 11, and 24. When such a battery is employed, sheets may be layered to place the cells in series, and an effective compromise between number of sheets and surface areas of sheets is to layer them in a diagonal as shown somewhat schematically in Fig. 2. Of course, battery selection would ultimately depend on such voltage and current densities required for a specific application, and time of discharge.

Layered above battery 28 would be another contact 32 which could be similar in construction to that of contact 26 an connected electrically to the opposite side of battery 28.

Cover 12 which encloses all of the layers of applicator 10 is made from a flexible conductive plastic material such as a polymer impregnated with carbon or surface metalized plastic. Insulating material 34 fills the space between the side wall of raised portion 14 and the various layers contained therein.

An electrically conductive adhesive material 36 coats the underside of lip 16 so that applicator 10 may be placed on and adhere to skin 18 and make good electrical contact.

It will be seen that the above described arrangement forms a complete electric circuit from one side of battery 28, to cover 12, adhesive material 36, skin 18, microporous membrane 22, liquid reservoir 24, and back to battery 28.

For a more particular description of the electrical circuit formed by the arrangement just described, reference is made to Fig. 3 wherein the circuit is shown schematically with numerals corresponding to the structure shown in Figs. 1 and 2.

Battery 28 is connected through contact 32, cover 12, and adhesive layer 36 to skin 18. The other side of battery 28 is connected electrically through contact 26, liquid reservoir 24 and membrane 22 to skin 18 to complete the circuit. Resistor Reff represents the effective resistance of the complete circuit, including skin 18, the adhesive layer 36, cover 12, battery 28 and its contacts 26 and 32, as well as reservoir 24 and membrane 22, In a system of this type, one of the aims is to establish a very low rate of current flow so that the medicament will be deposited slowly over a long period of time. Current flow of down as low as 0.0001 ampere–hour per square centimeter of skin surface below membrane 22 is a typical current which may be selected for the application of a particular drug. Electrical resistance of the skin to current flow is of the order of 6–9 K ohms and is roughly independent of the distance between the points on the skin where electrical contact is made. This is because skin electrical resistance is largely that of resistance to penetration, the current flowing through the fluids of the body in which electrical resistance being very low. Thus, in order to establish current flow at the rate indicated, by ohm's law, it is seen that total resistance of the circuit using a 1.5 volt battery should be about 360 K ohms for each square centimeter of application. This resistance, the effective resistance, Reff, of the circuit, can be built into any one component or combination of components of the circuit shown in Fig. 3, including the battery resistance, electrodes, cover material, etc. In addition, if desired, in order to maintain current flow constant over the full period of operation a constant current limiting device can be made integral with and a part of conductor 26, or any other part of the circuit where it is found convenient to do so.

Furthermore, as indicated schematically in Fig. 4, applicator 10 may be designed to incorporate provision to insure that the deposit of medicament will cease after a given period of time or after a certain quantity of drug is administered. This can be accomplished by inserting in the circuit an integrating device such as a reverse plating cell 38. Cell 38, as is known in the art, comprises a pair of electrodes on which one is a coating of material to be transferred to the other electrode. When all of the plating material is deposited, after a predetermined period of time based upon the thickness of the original coating has lapsed, or integrated current flow representing the desired quantity of drug to be delivered, there is a large increase in internal resistance resulting in a substantial drop of current flow and an effective halt to drug migration. Such a device can be employed to establish in advance the period of time over which the medicament is to be applied or, as noted above, the quantity of the drug to be delivered. Cell 38 is a relatively high resistance device and could provide for much of the high resistance required for the operation of applicator 10.

Cell 38 may be made a part of contact 32 or be inserted between contact 32 and cover material 14. In addition, provision may be made for current flow to be built up gradually to avoid any shock to the recipient of the drug.

Applicator 10 may be prepared in advance, in different sizes and shapes, sealed within a plastic pouch, with a protective strip over its exposed side. Different drugs can be incorporated for particular applications, batteries may be varied to meet specific current flow requirements, and of course the electrical orientation of each battery would depend on the particular medicament. In the use of the device, the protective strip is removed and the applicator placed on the skin where desired and current flow starts immediately along with migration of the drug.

With the drug applicators and electrode constructions of the present invention, at least two drugs can be transdermally transported simultaneously, across the skin, using one or more transfer modalities. Such an arrangement may be particularly effective in situations where two or more drugs work better together than if taken spearately. For example, aspirin and codei exhibit a synergistic or improved effect when used together, rather than independently of each other. Other well known drug combinations, such as Dristan exhibit similar results. Thus,with the applicators of the present invention, drug delivery can be varied and may be effected by one or more mass transfer phenomena such as electroosmosis and iontophoresis and electrophoresis.

It should be recognized that both mass transfer processes require an electric power source, and in the case of electrophoresis an ionized drug migrates from the applicator patch through the skin and into the blood stream, whereas in the case of electroosmosis, a fluid carrier, such as water is likewise transported across the skin and into the blood stream carrying along with it any and all dissolved constituents (ionized drugs or otherwise). Either or both of these two physicochemical phenomena may jointly work together or independently in transdermally carrying a drug or drugs across the skin in a desired dosage release and relatively steady pattern.

It should also be noted that as a convenience like numerals are representative of similar elements common to the various embodiments of the invention.

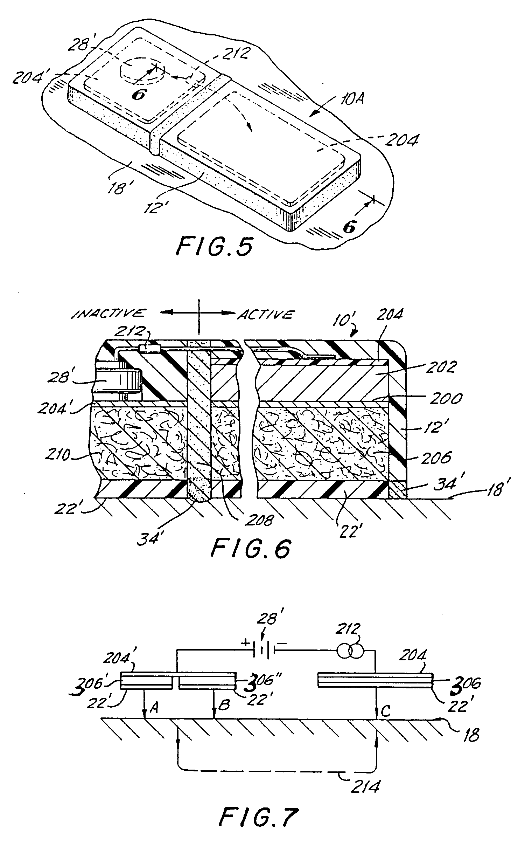

Referring now to Figures 5 − 6, a side by side patch or applicator 10A construction is illustrated affixed to the skin 18. As shown therein, element 10' represents the active electrode and it is spaced apart or separated from the inactive electrode by a suitable gap or space, including an air gap. As best shown in Figure 6, the gap is suitably illustrated as a "dam" 208 which may be made of an inpervious, non − conductive material, such as silicone. This dam 208 maintains electrode separation and provides a seal against the skin so as to preclude any "shorting"effect across the electrodes which might occur due to sweat and other moisture accumulated on the surface of the skin beneath or adjacent such dam or electrode barrier means. Element 12' is a cover material similar to that outer cover 12 of Figures 1 − 2, although it need not be electrically conductive, since the power source or battery 28' and series connected constant current device 212 (which may suitably comprise a diode) is suitably connected by wire leads or conductors to the separate conductive terminals or film elements 204 and 205'. Preferred elements may be made of a carbonized plastic, foil or other conductive film, such as a metalized mylar.

Membrane 22' is optional as is element 22 of Figures 1 − 4 and comprises a semi − permeable, microporous membrane element having an adhesive and preferably gel − like quality.

Element 34' is a suitable impermeable, insulating material which is preferibly formed so as to protrude beyond element 22' and thus form an effective dam − like seal between the separated electrodes forming the applicator patent. Thus, element 34' should be impervious to not only the drugs, water, etc., but it should also be non − conducting.

The "indifferent" electrode 210 element 210 which may also comprise a drug matrix or reservoir is disposed between microporous membrane 22' and the conductive element 204'. On the "active" electrode side of the applicator patch 10A, there is disposed suitable drug matrix or reservoir 206, such as an electrolyte solution of low concentration between the nonporous membrane 22' and preferably another semi − permeable membrane 200, so as to provide for a further (upper) high concentration drug matrix or

EP 0 240 593 B1

reservoir 202. Such an arrangement for the "active" electrode side of the patch facilitates maintaining a predetermined gradient concentration and desired pH which all aid in providing optimum drug administration.

In Figure 7, which represents an electrical schematic of the invention, numeral 214 (shown in phantom) is current flow generated by the power source or battery 28'. Additionally, this figure illustrates schematically an even further applicator construction or modification comprising three distinct electrodes, all of which are "active" electrodes of similar internal construction, and they are identified by numerals 306, 3 06' and 306''. Reference arrow A, for example, may be employed to transdermally deliver a drug by means of electroosmosis,whereas reference arrows B and C may be employed to deliver transdermally positive ions and negative ions, respectively, by means of iontophoresis or electrophoresis.

It should also be appreciated that electroosmosis moves fluid away from the positive to the negative electrode and such mass transfer process is not very dependent upon concentration levels of the drug. On the other hand, electrophoresis takes place at either the positives or negative electrodes and generally requires a low concentration and high dilution as well as a controlled pH.

Accordingly, as noted hereinabove, although Figures 5 – 6 show different electrode constructions, both sides can be of similar construction, and thus both sides would then comprise "active" electrodes in contrast to, as shown, where the left side being an inactive electrode and the right side being an active electrode. With such applicator constructions, it will be appreciated that one or both halves could deliver iontophoretic drugs or one side of the patch could delivery a drug iontophoretically and the other patch side could deliver a drug electroosmotically.

It should be noted, and as is clearly illustrated in Figure 5, the electrodes need not necessarily be of the same size (or even shape) although they may be conveniently manufactured to the same size and/or configuration. In a like manner, it will be recognized that these electrodes and applicator constructions in effect do not really incorporate a true "lip" area or skin electrode as does the device of Figures 1 – 4, although the element 34' still serves as a tacky sealing boundry about the periphery of the applicator, as does element 34 of Figure 2.

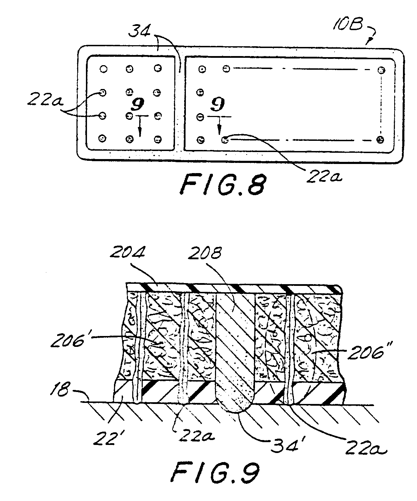

In Figures 8 – 9, there is shown an alternate construction of yet another suitably unequally sized, electrode configurations, wherein the applicator 10B includes a plurality of randomly placed or preferably uniformly aligned rows of a high tack, impermeable gel 22a. Such an adhesive gel aids in retaining the patch on one's skin and inasmuch as some areas of the patch would thus be more tacky than other areas of the patch, such construction enables the manufacture of a patch wherein the adhesive gel 22' has less adhesive strength than that of the gel 22a. By this arrangement, the patch may be easily removed from the skin and replaced thereon should one require removal and replacement of the same patch, say for example, upon bathing or showering, or even pursuant to a physician's instructions where it is desired to transmit a drug dosage periodically in lieu of a continuous steady state condition.

As best shown in Figure 9, the drug matrixes or reservoirs of the electrodes are represented by reference numerals 206' and 206'', and same are separated by a suitable silicone barrier dam 208. The high tack zones or areas 22a are generally of a harder gel than that gel of 22'. Thus, these elements 22a which may be either of elongated fiber – like construction or of stubby spherical shape (not shown) they both serve the same identical purpose. However, as one construction may be easier or less expensive to fabricate than the other, neither one is more preferable than the other.

In a series of tests conducted using applicators made generally in accordance with that design exemplified by the "inactive" electrode shown in Figure 6, using drugs such as testosterone, tobramycin, and aspirin, the following tabled results were obtained from sampled blood and urine of rabbits which formed the test animals in all cases. The control represented patches or applicators without any power source, but containing drug. Note that the first two drugs were radiotagged, whereas aspirin was tested both as radiotagged and conventional or untagged, and the samples taken were assayed for the total radioactive count.

Albino rabbits were employed for the tests, and each rabbit weighed at least six pounds. (Note: rabbits do not have sebaceous glands). The back of each rabbit's neck was shaved to permit better contact of the applicators and the skin or epidermis. The applicators were secured in place by an elastic bandage.

Each applicator was of the same size (3" x 4") and equally divided, with one half being the cathodic or negatively charged electrode, and the other half being the anode or positively charged electrode; and the target drug was loaded equally into both halves of the applicator. Only one drug was employed in each test. The target drug was also dissolved in distilled water and 4 ml of the resultant solution was loaded into the patches.

The blood samples were secured prior to initiation of drug delivery, and at hourly intervals for the next six hours. The urine samples were occasionally collected to determine if the drug was being metabolized

5

and eliminated from the animals during the course of the experiments. Where more than one series of tests were employed using the same groups of animals, at least 36 hours elapsed between the end of one test series and the beginning of a second test in order to insure that there was no interference between each test series. In one series of tests, the patches were without electrical power, and in another series of tests, the patches were powered by table top power supplies for purposes of convenience and expediency.

TABLE I

Testosterone Drug Delivery

The control series, with no applied power, did not yield any levels of radioactivity in the sampled blood.  When the table top battery supply was connected to the patch, the following results were obtained.

| TIME | Counts per minute | |
| | Powered Patch | Unpowered Patch |
| --- | --- | --- |
| 0 h | 0 | 0 |
| 1 h | 32 | 0 |
| 2 h | 67 | 0 |
| 3 h | 71 | 0 |
| 4 h | 78 | 0 |
| 5 h | 81 | 0 |
| 6 h | 88 | 0 |

n=29 rabbits
6 series of tests

The urine was collected into two batches. The first three hours of pooled urine per animal exhibited no radioactivity (less than one count per minute). The second three hours average radioactivity was 7 dpm.

TABLE II

Trobramycin Drug Delivery

There was no noticeable transdermal delivery of trobramycin without applied power. The results over the six hours of testing ranged from 0 to 2 disintegrations per minute. When the table top power supply was applied to the patch, the results were noticeably different, as is seen hereinbelow.

|  | Counts per minute | |
| TIME | Powered Patch | Unpowered Patch |
| 0 h | 0.2 | 0.1 |
| 1 h | 10 | 0 |
| 2 h | 19 | 0.5 |
| 3 h | 27 | 0.1 |
| 4 h | 35 | 0.2 |
| 6 h | 40 | 0.2 |

n= 9 rabbits
2 test series

The urine samples demonstrated no radioactivity during the first three hours. The average radioactivity in the subsequent period was 2.5 dpm.

TABLE III

Aspirin Drug Delivery

As radioactively tagged aspirin was not obtainable, thus liquid aspirin was employed and assayed using conventional methods. The results for the powered delivery were as follows.

---

| TIME | Aspirin Level |
|------|---------------|
| 0 h | 0 |
| 2 h | 5 |
| 4 h | 23 |
| 6 h | 57 |

n=14 rabbits
2 test series

---

It is clear from these tests that due to the migration phenomenon drugs can be transported transdermally using an electrically powered applicator, and that desired drug levels may be obtained by environmental optimization, i.e. pH, drug loading, current density, etc.

It will also be appreciated that since both ionic and non-ionic drugs can be transdermally delivered, it may not be necessary to ionically charge a non-ionic drug, should it be desired to transdermally transport such a drug by an electrically powered applicator patch.

Also, the applicators may be made with the same electrode configurations or with different designs, such as those constructions described herein. It is also within the scope of the present invention to have an applicator patch embodying three different electrode structures, such as that described with reference to Figure 7. Furthermore, although both an active and an inactive electrode are required for use with an electrophoresis mass transfer drug system, all of the electrodes may be active in those systems employed where either electroosmosis or both electroosmosis and electrophoretic systems are utilized to deliver one or more drugs into the blood stream.

**Claims**

1. A transdermal drug applicator (10, 10A, 10B) for application to a living body for the migration of at least one medicament through the skin (18) into the blood stream comprising:
   at least two electrode elements (210) forming said applicator separated from each other by non-conductive and impervious barrier means (208, 34), said electrode elements comprising:
   reservoir means (24, 202, 206, 206', 206") in at least one of said electrode elements (210) of said applicator (10, 10A, 10B), for contacting the skin (18) and a conductive element (204, 204'), said reservoir means (24, 202, 206, 206', 206") containing said medicament,
   a circuit including a power source (28, 28') for supplying electric power to said conductive elements (204, 204') and said reservoir means (24, 202, 206, 206', 206"),
   cover means (12, 12') partially enclosing said electrode elements, said cover means (12, 12') comprising adhesive means (36) for affixing said applicator (10, 10A) to said skin (18), whereby an electrical circuit through the skin (18) is formed when said applicator (10, 10A, 10B) is affixed to said skin (18), thereby creating at least one physico/chemical mass transfer phenomena which causes said at least one medicament to migrate through the skin (18),

8

characterized in that the reservoir means (202, 206, 206', 206'') contains a plurality of high tack areas (22a) for retaining the applicator on the skin (18).

2. A transdermal drug applicator according to claim 1, wherein said high tack areas (22a) are of elongated fiber – like construction.

3. A transdermal drug applicator according to claim 1 or 2, wherein said high tack areas (22a) are of stubby spherical shape.

4. A transdermal drug applicator according to one of the claims 1 – 3, wherein said reservoir means is a gel (22) and said high tack areas are generally a harder gel (22a) than that of said reservoir means (202, 206).

5. A transdermal drug applicator according to one of the claims 1 – 4, wherein said high tack spots (22a) comprise an adhesive gel of greater tackiness as compared to the adhesive means which affixes said applicator to said skin (18).

6. A transdermal drug applicator according to one of the claims 1 – 5, wherein said high tack areas (22a) are randomly spaced, and reinforce said drug applicator (10, 10A, 10B).

7. A transdermal drug applicator according to one of the claims 1 – 5, wherein said high tack areas (22a) are uniformly spaced, and reinforce said drug applicator (10, 10A, 10B).

8. A transdermal drug applicator according to one of the claims 1 – 7, wherein each said electrode element (210) comprise reservoir means (202, 206) having a plurality of high tack spots (22a).

9. A transdermal drug applicator according to one of the previous claims wherein said electrode elements (210) are of the same construction.

10. A transdermal drug applicator according to one of the claims 1 – 8, wherein said electrode elements (210) are of different constructions.

11. A transdermal drug applicator according to one of the claims 1 – 10, wherein said electrode elements (210) are of different size.

12. A transdermal drug applicator according to one of the claims 1 – 11, wherein said electrode elements (210) comprise reservoir means (202, 206) and adjacent conductive elements (220), and said circuit being connected to said conductive elements (220) so as to enable said at least one physico/chemical mass transfer phenomena to take place at the surface of the skin (18).

13. A transdermal drug applicator according to one of the previous claims, wherein said drug applicator (10) further including conductive elements (260) embedded in an inert mass (250a) of material disposed atop said drug applicator (10, 10A) with a reverse plating cell (38, 38') disposed therebetween, and said reverse plating cell (38, 38') enabling a different drug delivery sequence or a different drug delivery rates where a plurality of drugs are administered from said drug reservoir means of said electrode elements (210).

14. A transdermal drug applicator according to claim 13, wherein said conductive elements (260) in said mass of inert material (250a) comprise carbonaceous material for providing an electrical current limiting effect and threshold above which current density at the skin (18) surface cannot increase.

15. A transdermal drug applicator according to one of the previous claims including a third electrode insaid applicator (10, 10A, 10B).

16. A transdermal drug applicator according to one of the previous claims wherein at least one of said electrodes constitutes an inactive or indifferent electrode (210).

**17.** A transdermal drug applicator according to one of the previous claims, wherein all of said electrodes are active electrodes.

**18.** A transdermal drug applicator according to one of the previous claims, wherein both ionic and non‒ionic drugs are transdermally delivered by means of said active electrodes.

**19.** A transdermal drug applicator according to one of the previous claims, wherein said electrodes (210) contain different drugs.

**Patentansprüche**

**1.** Transdermaler Medikamentenapplikator (10, 10A, 10B) zur Anwendung an einem lebenden Körper für die Migration zumindest eines Medikaments durch die Haut (18) in den Blutstrom, welcher aufweist:
zumindest zwei den Applikator bildende Elektrodenelemente (210), die voneinander durch nichtleitende und undurchlässige Barrieremittel (208, 34) getrennt sind, wobei die Elektrodenelemente folgendes aufweisen:
Behältermittel (24, 202, 206, 206', 206'') in zumindest einem der Elektrodenelemente (210) des Applikators (10, 10A, 10B) für den Kontakt zur Haut (18) und einem leitfähigen Element (204, 204'), wobei das Behältermittel (24, 202, 206, 206', 206'') das Medikament enthält,
einen Schaltkreis mit einer Energiequelle (28, 28'), um die leitfähigen Elemente (204, 204') und das Behältermittel (24, 202, 206, 206', 206'') mit elektrischer Energie zu versorgen,
ein Abdeckmittel (12, 12'), welches die Elektrodenelemente teilweise umschließt, wobei das Abdeck‒mittel (12, 12') Haftmittel (36) zum Befestigen des Applikators (10, 10A, 10B) an der Haut (18) aufweist, wodurch durch die Haut (18) ein elektrischer Schaltkreis gebildet wird, wenn der Applikator (10, 10A, 10B) an der Haut befestigt ist, wobei zumindest ein physikalisch chemisches Transportphänomen hervorgerufen wird, welches verursacht, daß dieses zumindest eine Medikament durch die Haut (18) wandert,
**dadurch gekennzeichnet, daß** das Behältermittel (202, 206, 206', 206'') eine Mehrzahl sehr klebriger Bereiche (22a) enthält, um den Applikator an der Haut (18) zu befestigen.

**2.** Transdermaler Medikamentenapplikator nach Anspruch 1, bei welchem die klebrigen Bereiche (22a) längtich‒faserartig ausgebildet sind.

**3.** Transdermaler Medikamentenapplikator nach Anspruch 1 oder 2, bei welchem die klebrigen Bereiche (22a) eine stumpfe sphärische Form besitzen.

**4.** Transdermaler Medikamentenapplikator nach einem der Ansprüche 1 bis 3, bei welchem das Behäl‒termittel ein Gel (22) ist und die klebrigen Bereiche im allgemeinen ein härteres Gel (22a) als jenes des Behältermittels (202, 206) ist.

**5.** Transdermaler Medikamentenapplikator nach einem der Ansprüche 1 bis 4, bei welchem die klebrigen Punkte (22a) im Vergleich zu dem Haftmittel ein haftendes Gel mit höherer Klebrigkeit enthalten, welches den Applikator an der Haut (18) hält.

**6.** Transdermaler Medikamentenapplikator nach einem der Ansprüche 1 bis 5, bei welchem die klebrigen Bereiche (22a) willkürlich verteilt sind und den Medikamentenapplikator (10, 10A, 10B) versteifen.

**7.** Transdermaler Medikamentenapplikator nach einem der Ansprüche 1 bis 5, bei welchem die klebrigen Bereiche (22a) gleichmäßig verteilt sind und den Medikamentenapplikator (10, 10A, 10B) versteifen.

**8.** Transdermaler Medikamentenapplikator nach einem der Ansprüche 1 bis 7, bei welchem jedes Elektrodenelement (210) ein Behältermittel (202, 206) aufweist, welches eine Mehrzahl von klebrigen Punkten (22a) enthält.

**9.** Transdermaler Medikamentenapplikator nach einem der vorigen Ansprüche, bei welchem die Elektro‒denelemente (210) gleich aufgebaut sind.

**10.** Transdermaler Medikamentenapplikator nach einem der Ansprüche 1 bis 8, bei welchem die Elektro‐ denelemente (210) unterschiedlich aufgebaut sind.

**11.** Transdermaler Medikamentenapplikator nach einem der Ansprüche 1 bis 10, bei welchem die Elektro‐ denelemente (210) unterschiedliche Größe besitzen.

**12.** Transdermaler Medikamentenapplikator nach einem der Ansprüche 1 bis 11, bei welchem die Elektro‐ denlemente (210) Behältermittel (202, 206) und benachbarte leitfähige Elemente (220) enthalten und der Schaltkreis an die leitfähigen Elemente (220) angeschlossen wird, um zu ermöglichen, daß dieses zumindest eine physikalisch chemische Massentransportphäomen an der Oberfläche der Haut (18) stattfindet.

**13.** Transdermaler Medikamentenapplikator nach einem der vorigen Ansprüche, bei welchem der Medika‐ mentenapplikator (10) weiters in einer inerten Masse (250a) eines Materials eingebettete leitfähige Elemente (260) enthält, die an der Oberseite des Medikamentenapplikators (10, 10A) angeordnet sind, wobei dazwischen eine Umkehrbeschichtungszelle (38, 38') angeordnet ist und diese Umkehrbe‐ schichtungszelle (38, 38') eine unterschiedliche Medikamentenzuführsequenz oder unterschiedliche Medikamentenzuführraten ermöglicht, falls eine Mehrzahl von Medikamenten aus dem Medikamenten‐ behältermittel der Elektrodenelemente (210) verabreicht wird.

**14.** Transdermaler Medikamentenapplikator nach Anspruch 13, bei welchem die leitfähigen Elemente (260) in der Masse (250a) aus inertem Material kohlenstoffhältiges Material enthalten, um einen Strombe‐ grenzungseffekt und eine Schwelle bereitzustellen, über welche die Stromdichte an der Hautoberfläche (18) nicht ansteigen kann.

**15.** Transdermaler Medikamentenapplikator nach einem der vorigen Ansprüche, bei welchem in dem Applikator (10, 10A, 10B) eine dritte Elektrode enthalten ist.

**16.** Transdermaler Medikamentenapplikator nach einem der vorigen Ansprüche, bei welchem zumindest eine der Elektroden eine inaktive oder neutrale Elektrode (210) bildet.

**17.** Transdermaler Medikamentenapplikator nach einem der vorigen Ansprüche, bei welchem alle Elektro‐ den aktive Elektroden sind.

**18.** Transdermaler Medikamentenapplikator nach einem der vorigen Ansprüche, bei welchem mittels der aktiven Elektroden sowohl ionische als auch nichtionische Medikamente transdermal zugeführt werden.

**19.** Transdermaler Medikamentenapplikator nach einem der vorigen Ansprüche, bei welchem die Elektro‐ den (210) unterschiedliche Medikamente enthalten.

**Revendications**

**1.** Applicateur (10,10A,10B) pour médicament transdermique à appliquer à un corps vivant pour faire migrer au moins un médicament à travers la peau (18) dans le circuit sanguin comprenant :
- au moins deux éléments d'électrode (210) formant l'applicateur, séparés l'un de l'autre par des moyens non conducteurs et imperméables (208,34) faisant barrière, les éléments d'électrode comprenant :
- des moyens faisant réservoir (24, 202, 206, 206',206'') dans au moins un des éléments d'électrode (210) de l'applicateur (10,10A,10B) venant en contact avec la peau (18) et un élément conducteur (204,204'), les moyens faisant réservoir (24,202,206,206',209'') contenant le médica‐ ment,
- un circuit électrique comprenant une source d'énergie (28,28') pour alimenter en énergie électrique les éléments conducteurs (204,204') et les moyens faisant réservoir (24,202,206,206',209''),
- des moyens faisant couvercle (12,12') renfermant partiellement les éléments d'électrode, ces moyens faisant couvercle (12,12') comprenant des moyens adhésifs (36) pour fixer l'applicateur (10,10A) sur la peau (18), un circuit électrique étant formé à travers la peau (18) quand l'applicateur (10,10A,10B) y est fixé, ce qui crée au moins un phénomène de transfert de masse physico/chimique faisant migrer au moins un médicament à travers la peau (18), caractérisé en ce que les moyens faisant réservoir (202,206,206',206'') contiennent une pluralité de zones d'attache forte (22a) pour retenir l'applicateur contre la peau.

2. Applicateur pour médicament transdermique selon la revendication 1, dans lequel les zones à haute adhésion (22a) ont une construction allongée en fibres.

3. Applicateur pour médicaments transdermique selon la revendication 1 ou 2, dans lequel les zones à haute adhésion (22a) ont une forme sphérique tronquée.

4. Applicateur pour médicament transdermique selon l'une des revendications 1 à 3, dans lequel les moyens faisant réservoir consistent en un gel (22) et les zones à haute adhésion consistent généralement en un gel (22a) plus dur que ceux des moyens faisant réservoir (202,206).

5. Applicateur pour médicament transdermique selon l'une des revendications 1 à 4, dans lequel les zones à haute adhésion (22a) comprennent un gel adhésif collant plus que les moyens adhésifs qui fixent l'applicateur à la peau (18).

6. Applicateur pour médicament transdermique selon l'une des revendication 1 à 5, dans lequel les zones à haute adhésion (22a) sont espacées au hasard et renforcent l'applicateur (10A,10B) de médicament.

7. Applicateur pour médicament transdermique selon l'une des revendications 1 à 5, dans lequel les zones à haute adhésion (22a) sont espacées uniformément et renforcent l'applicateur (10A,10B) de médicament.

8. Applicateur pour médicament transdermique selon l'une des revendications 1 à 7, dans lequel l'élément d'électrode (210) comprend des moyens faisant réservoir (202,206) ayant une pluralité de zones à haute adhésion (22a).

9. Applicateur pour médicament transdermique selon l'une des revendications précédentes, dans lequel les éléments d'électrodes (210) sont d'une même construction.

10. Applicateur pour médicament transdermique selon l'une des revendications 1 à 8, dans lequel les éléments d'électrodes (210) sont d'une construction différente.

11. Applicateur pour médicament transdermique selon l'une des revendications 1 à 10, dans lequel les éléments d'électrodes (210) sont d'une taille différente.

12. Applicateur pour médicament transdermique selon l'une des revendications 1 à 11, dans lequel les éléments d'électrode (210) comprennent les moyens faisant réservoir (202,206) et des éléments conducteurs adjacents (220), le circuit étant connecté aux éléments conducteurs (220) de façon à permettre au moins un phénomène de transfert de masse physico/chimique à la surface de la peau (18).

13. Applicateur pour médicament transdermique selon l'une des revendications précédentes, dans lequel l'applicateur (10) comprend en outre des éléments conducteurs (260) noyés dans une masse inerte (250a) d'un matériau disposé au sommet de l'applicateur (10,10A) avec une cellule à plaque inverse (38,38') interposée, cette cellule à plaque inverse (38,38') permettant une séquence différente de délivrance ou des taux de délivrance différents des médicaments quand une pluralité de médicaments est administrée depuis les moyens des éléments d'électrode(210) faisant réservoir.

14. Applicateur pour médicament transdermique selon la revendication 13, dans lequel les éléments conducteurs (260) noyés dans la masse inerte du matériau (250a) comprennent un matériau carbonaté permettant un effet de seuil limitant le courant électrique, au-dessus duquel la densité de courant sur la surface de la peau (18) ne peut pas augmenter.

15. Applicateur pour médicament transdermique selon l'une des revendications précédentes, comprenant une troisième électrode dans l'applicateur (10,10A,10B).

**EP 0 240 593 B1**

16. Applicateur pour médicament transdermique selon l'une des revendications précédentes, dans lequel au moins une des électrodes constitue une électrode inactive ou indifférente (210).

17. Applicateur pour médicament transdermique selon l'une des revendications précédentes, dans lequel toutes les électrodes sont des électrodes actives.

18. Applicateur pour médicament transdermique selon l'une des revendications précédentes, dans lequel à la fois des médicaments ioniques et non ioniques sont délivrés par voie transdermique au moyen des électrodes actives.

19. Applicateur pour médicament transdermique selon l'une des revendications précédentes, dans lequel les électrodes (210) contiennent des médicaments différents.

# FIG.1

# FIG.2

# FIG.3

# FIG.4

FIG.5

INACTIVE ← → ACTIVE

FIG.6

FIG.7

34    10B

22a

*9*    *9*

22a

FIG.8

204    208

206'    206"

18

22'    22a    34'    22a

FIG.9